# EUROPEAN PATENT SPECIFICATION

(11) **EP 1 845 856 B1**
(45) Date of publication and mention of the grant of the patent: **13.03.2019**
(21) Application number: 06710765.6
(22) Date of filing: 26.01.2006
(51) Int. Cl.: A61B 8/02

(54) **METHOD AND SYSTEM FOR DERIVING A HEART RATE WITHOUT THE USE OF AN ELECTROCARDIOGRAM IN NON-3D IMAGING APPLICATIONS**
VERFAHREN UND SYSTEM ZUR ERMITTLUNG EINER HERZFREQUENZ OHNE VERWENDUNG EINES ELEKTROKARDIOGRAMMS BEI NICHTDREIDIMENSIONALEN ANWENDUNGEN
METHODE ET SYSTEME DE MESURE DU RYTHME CARDIAQUE SANS ELECTROCARDIOGRAMME DANS DES APPLICATIONS D'IMAGERIE NON EN 3D

(30) Priority: 31.01.2005 US 648484 P
(43) Date of publication of application: 24.10.2007
(73) Proprietor: Koninklijke Philips N.V., 5656 AE Eindhoven (NL)
(72) Inventor: CLARK, David, W., Briarcliff Manor, NY 10510-8001 (US)
(74) Representative: de Haan, Poul Erik
(86) International application number: PCT/IB2006/050293
(87) International publication number: WO 2006/079992

(56) References cited:
- JP-A- 2000 225 115
- US-A- 5 442 940
- US-A1- 2003 016 782
- US-A1- 2005 049 503
- US-B1- 6 312 382
- YAMAKOSHI Y ET AL: "FOETAL HEART RATE IN EARLY PREGNANCY BY DETECTION OF OPTIMALLY ESTIMATED DISPLACEMENTS WITH PULSED DOPPLER SIGNALS" ULTRASONICS, IPC SCIENCE AND TECHNOLOGY PRESS LTD. GUILDFORD, GB, vol. 32, no. 5, 1 September 1994 (1994-09-01), pages 391-395, XP000474452 ISSN: 0041-624X

## Description

The present invention relates generally to medical devices. More particularly, the present invention relates to a method and device for deriving a heart rate without the use of an electrocardiogram in non-3D imaging applications.

Medical ultrasound imaging has become a popular means for visualizing and medically diagnosing the condition and health of interior regions of the human body. With this technique, an acoustic transducer probe, which is attached to an ultrasound system console via an interconnection cable, is held against the patient's tissue by the sonographer where it emits and receives focused ultrasound waves in a scanning fashion. The scanned ultrasound waves, or ultrasound beams, allow the systematic creation of image slices of the patient's internal tissues for display on the ultrasound console. The technique is generally quick, painless, fairly inexpensive and safe, even for such uses as fetal imaging.

Ultrasound imaging systems commonly in use generate and transmit ultrasound signals to map internal tissue typography, vascular fluid flow rates, and abnormalities. The systems typically incorporate several methods, or modes, of imaging, i.e. Brightness Mode (B-Mode), Harmonic, Spectral Doppler, and Color Flow.

Each imaging method has its characteristic uses and limitations. B-Mode imaging is typically used to image the structure of internal tissue and organs with high spatial resolution. Generally to achieve this degree of spatial resolution, short-duration ultrasound pulses are advantageous. Harmonic imaging uses the harmonic frequencies produced from nonlinear wave propagation. Harmonic imaging can reduce clutter, sidelobes, and aberration compared to the more traditional fundamental B-mode imaging, but typically involves compromising spatial resolution.

Color Flow imaging is primarily used to image blood flow and locate abnormal or turbulent flows within the cardiovascular system. Color Flow images are usually overlaid on to a B-Mode structural image. However, the ultrasound properties necessary for proper Color Flow imaging differ from those used in B-Mode. Color Flow imaging requires multiple pulses to detect motion, and longer duration ultrasound pulses than commonly used for B-Mode scans for sensitivity. Low ultrasound pulse repetition rates are desirable for slow-flowing veins, but for the faster flows found in the arteries and heart, higher ultrasound pulse repetition rates are necessary to properly avoid aliasing errors.

Spectral Doppler uses a very large number of ultrasound pulses (or a continuous wave) in the same direction, and converts the resulting echo data stream into a frequency spectrum versus time display and an audio output. Spectral Doppler provides more detailed blood flow dynamics information for one location, in contrast to Color Flow's simple estimation for many locations. Typically Color Flow is used to decide where to place the Spectral Doppler sample location.

3D ultrasound imaging involves scanning the ultrasound pulses over a volume rather than one plane, either mechanically or electrically. Typically the volume is scanned as a series of 2D planes. The echo data is usually displayed either as a volume-rendered image or as 2D planar images sliced through the volume data. The name 4D imaging is sometimes used when 3D volumes are acquired and/or displayed rapidly enough to see motion of the structure being imaged (time being the fourth dimension).

The heart and the fetus have been the two main applications for 3D ultrasound imaging, because both involve significant volumes of liquid that are nearly transparent to ultrasound, so the anatomy can be visualized relatively easily in three dimensions.

Particularly with color flow imaging, a 3D acquisition is typically too slow for the fast motion of the heart, so for adult or pediatric 3D cardiac exams an electrocardiogram is used to synchronize the ultrasound acquisition over multiple cardiac cycles. An electrocardiogram is impractical for a fetal heart exam, however. Nelson, Sklansky, and Pretorius at the University of California at San Diego published a technique for deriving the fetal heart rate from the 2D B-mode images in a slow (many heart cycle) 3D scan through the fetal heart, then using the derived heart rate to shuffle the 2D images into 3D volumes of the fetal heart at multiple points of the cardiac cycle. That technique (called the NSP technique hereafter) is implemented in several commercially available ultrasound systems, and will be described in more detail below. The NSP technique has only been applied to post -processing 3D image acquisitions of the fetal heart, and it depends on having significant cardiac motion in the 2D B-mode images.

US 6,312,382 B1 discloses a method and apparatus for processing acoustic information obtained with an ultrasound device to generate cardiac information, such as, for example, a patient's heart rate. An ultrasound system is utilized to image a patient's heart to obtain acoustic information relating to the patient's heart. The acoustic information is converted into electrical information, which is then processed to extract a particular feature of interest therefrom. Processing of the electrical signals comprises the steps of generating a time-series from a series of image frames and then performing a spectral analysis on the time series. JP2000225115 discloses determining the heart rate baesd on processing the autocorrelation of the acoustic information relating to the patient's heart.

An objective of the present disclosure is to extend the NSP technique to derive, display or use the heart rate without needing an electrocardiogram, in situations other than 3D imaging of the fetal heart. A further objective of the present disclosure is to extend the NSP technique to operate on data other than 2D B-mode slices of a 3D acquisition, which is particularly useful in non-cardiac exams where there is very little cardiac-cycle motion in the B -mode images and electrocardiograms are seldom used. A further objective of the present disclosure is to extend the NSP technique to operate repetitively, including using overlapping time segments, to provide rapidly updated heart rate estimates in a live imaging situation.

The above-mentioned object is solved by a method according to claim 1 for determining a heart rate from a set of ultrasound images, in situations other than 3D imaging of a fetal heart. The estimated heart rate is the frequency of the peak of the summed power spectra of a subset of spatial points over a set of ultrasound images. The derived heart rate can be used to set the time span of a repetitive loop display to an integral number of heartbeats, or to combine data from multiple heartbeats for noise reduction or temporal resolution improvement, or to display the heart rate numerically in a live imaging situation, or to reconstruct a 3D or 4D volume in applications other than fetal heart. The heart rate can be derived from ultrasound data other than fetal cardiac elevation-swept B-mode images, such as M-mode, cardiac or arterial color flow correlations or velocities, or spectral Doppler data.

According to further aspects of the present invention, a system according to claim 4 and a computer readable storage medium according to claim 7 are provided.

An additional embodiment of the present disclosure provides for an ultrasound medical imaging system. The ultrasound medical imaging system includes an ultrasound imager having an ultrasound transducer, a processor, and a video display.

These and other features, aspects, and advantages of the present invention will become better understood with regard to the following description, appended claims, and accompanying drawings wherein:
FIG. 1 is a flow chart illustrating the steps for determining a heart rate from a set of ultrasound images in accordance with an embodiment of the present disclosure;
FIG. 2 is a schematic view illustrating an ultrasound imaging system in accordance with an embodiment of the present disclosure; and
FIG. 3 is a diagram illustrating the overlapping derivation of heart rate in a continual imaging situation.

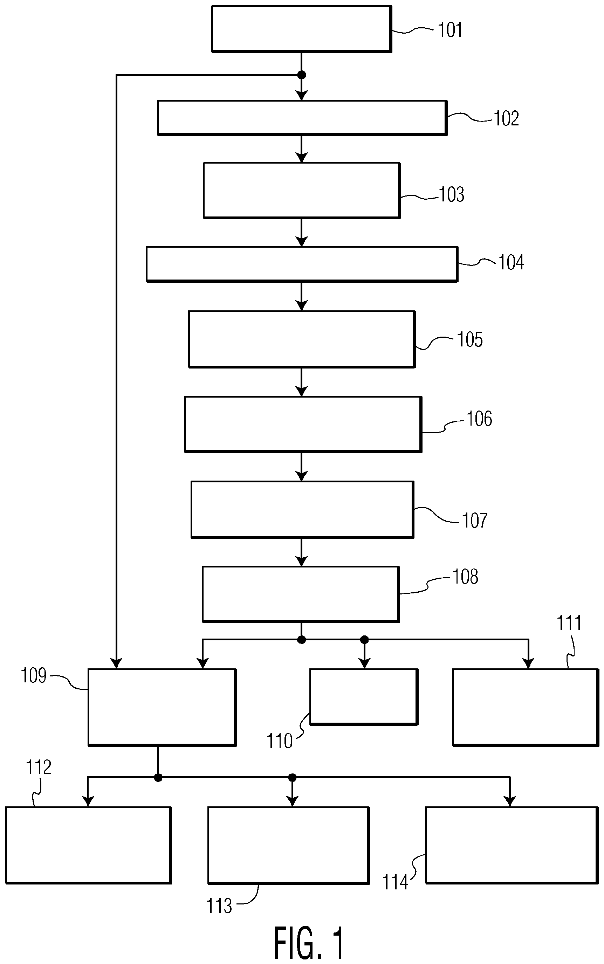

As shown in FIG. 1, an embodiment of the present disclosure provides a method for determining a heart rate from a set of ultrasound cardiac images. In step 101, an ultrasound imaging device begins a scan to acquire 2D cardiac images (later it will be described how this can be other than 2D cardiac images). Proceeding on, step 102 selects a subset of spatial points, such as a uniformly spaced grid. The selection step may be performed either manually or as an automated process. In step 103, the DC offset and slow variation is removed from each spatial point. Proceeding to step 104, the spatial points selected in step 102 are plotted with respect to time and a window function is applied to the data in step 105. Two appropriate window functions are the Hann and Hamming functions. A power spectrum is calculated in step 106 for the windowed data. In step 107, all the power spectra are summed, including both positive and negative frequencies. From the summed power spectra, a power spectrum peak is derived and processed along with the time sampling rate between image scans to determine the heart rate in step 108. The summed power spectrum covers the frequency range from zero to half the sample rate (the sample rate is the 2D frame rate). The location of the peak of the power spectrum is therefore at some fraction of the sample rate. Multiplying that by the sample rate in Hertz gives the heart rate in Hertz, and multiplying by 60 gives the heart rate in beats per minute.

There are several alternative ways that the derived heart rate may be used, as shown in Fig. 1. The prior art uses the heart rate to rearrange slowly elevation-swept 2D images of a fetal heart into multiple 3D volumes at different times of the heart cycle, step 109.

Other alternative uses of the derived heart rate, shown in Fig. 1, are a subject of the present disclosure. If the 2D images are elevation-swept, they may be rearranged into multiple 3D volumes in applications other than fetal heart. The length of a repetitive loop display of some subset of the 2D images (typically called a cine-loop) can be set to a whole number of cardiac cycles to minimize discontinuities when the loop wraps from end back to beginning, step 111. The reordered images from step 109 can be averaged to reduce variance and noise, step 113. The reordered images from step 109 can also be interleaved to improve temporal resolution, step 114.

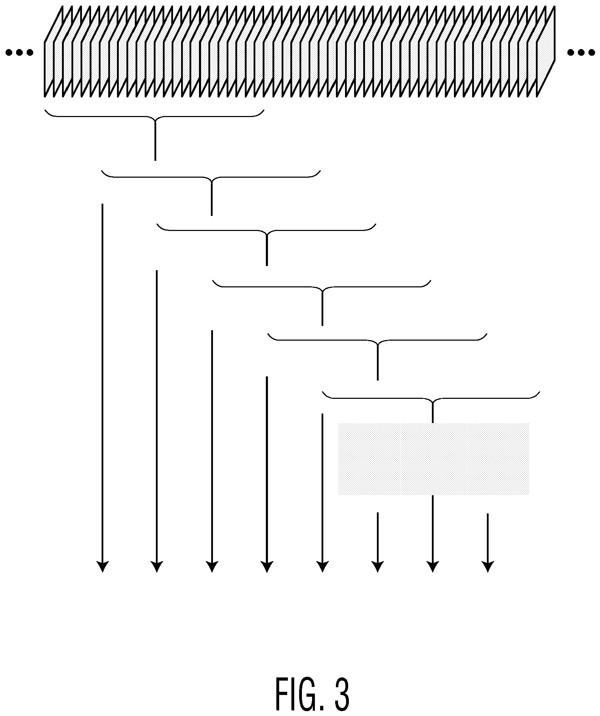

The derived heart rate may also be displayed numerically, which is particularly useful when it is done repetitively in a live imaging situation. To provide a more rapidly updating heart rate display while still using several heartbeats of data to derive the heart rate accurately, the time spans of data for each heart rate estimate may be overlapped, as shown in FIG. 3. For example, a 4 second time span can be stepped along every 0.5 second.

A further subject of the present disclosure is to use data other than fetal heart swept-elevation B-mode images to derive the heart rate. The data could be B-mode images of a non-fetal heart, or B-mode images of a fetal heart without elevation sweeping. The data could also be M-mode data, either grayscale or color. The data could also be color flow data of a heart or artery, either complex correlations or detected velocities. The data could also be Doppler spectra.

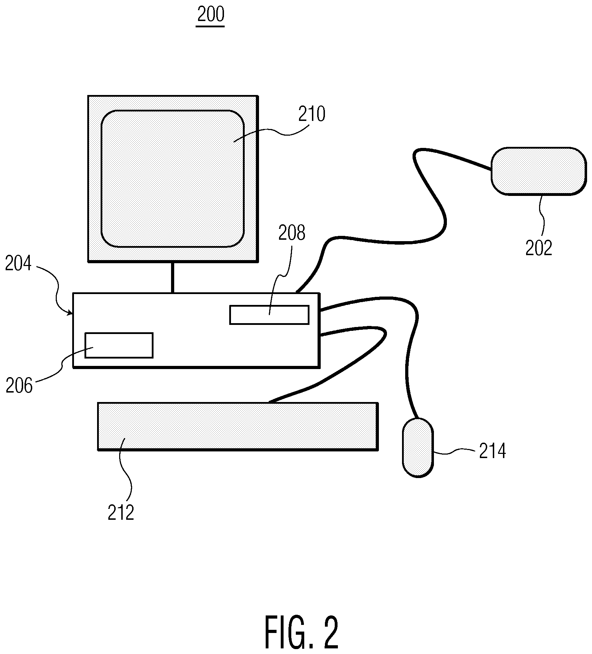

Another embodiment of the present disclosure, shown in FIG. 2, provides for a medical ultrasound imaging system 200. The system 200 includes an ultrasound transducer assembly 202 connected to an imaging workstation 204. The imaging workstation 204 contains one or more processors 206 and at least one storage device 208, such as a hard drive, RAM disk, etc. The storage device(s) 208 may be used for storing the controlling and imaging software for the ultrasound system 200 as well as providing temporary and long term storage of image data acquired by the ultrasound transducer 202. The ultrasound imaging system 200 also provides a video display 210 and user input devices, including a keyboard 212 and a mouse 214.

The processor 206 is configured to execute the controlling and imaging software. The imaging software allows the operator of the system 200 to visualize and manipulate the data received from the ultrasound transducer 202. Additionally, the imaging software includes subroutines to perform the method of the present disclosure as exemplified by FIG. 1 and described in detail above.

In short, the processor 206 executes a set of programmable instructions for performing the method of the present disclosure. It is contemplated that the set of programmable instructions can be stored within the at least one storage device 208 and/or on a computer-readable medium, such as a CD, 3.5" diskette, hard drive, etc., and capable of being executed by the processor 206.

The described embodiments of the present invention are intended to be illustrative rather than restrictive, and are not intended to represent every embodiment of the present invention. Various modifications and variations can be made without departing from the scope of the invention as set forth in the following claims both literally and in equivalents recognized in law.

## Claims

1. A method for determining a heart rate from a set of ultrasound images comprising the steps of:
acquiring (101) a set of ultrasound images;
selecting (102) a subset of spatial points from said set of ultrasound images;
plotting each of said selected spatial points with respect to time;
applying (104) a window function to window each of said selected spatial points with respect to time; and
processing (106) data corresponding to each of said selected spatial points, including computing a power spectrum for each of said selected spatial points windowed with respect to time,
**characterized by** the steps of:
summing (107) all power spectra; and
determining (108) a spectral peak frequency for said summed power spectra, wherein said determined spectral peak frequency is substantially identical to said heart rate.

2. The method according to Claim 1, further comprising the step of subtracting (103) a DC offset from each of said selected spatial points.

3. The method according to Claim 1, further comprising the step of displaying (110) said heart rate.

4. A system (200) for determining a heart rate from a set of ultrasound images comprising:
means (202) for acquiring a set of ultrasound images;
means (214) for selecting a subset of spatial points from said set of ultrasound images;
means for plotting each of said selected spatial points with respect to time;
means for applying a window function to window each of said selected spatial points with respect to time;
means (206) for processing data corresponding to each of said selected spatial points, including means for computing a power spectrum for each of said selected spatial points windowed with respect to time,
**characterized in that** the system (200) further includes:
means (206) for summing all power spectrum; and
means for determining a spectral peak frequency for said summed power spectra,
wherein said determined spectral peak frequency is substantially identical to said heart rate.

5. The system according to Claim 4, further comprising means for subtracting a DC offset from each of said selected spatial points.

6. The system according to Claim 5, further comprising means (210) for displaying said heart rate.

7. A computer readable medium storing a set of programmable instructions for being executed by a processor (206) for performing a method for determining heart rate from a set of acquired ultrasound images comprising the steps of:
selecting (102) a subset of spatial points from said set of ultrasound images;
plotting each of said selected spatial points with respect to time;
applying a window function to window each of said selected spatial points with respect to time;
processing (106) data corresponding to each of said selected spatial points, including computing a power spectrum for each of said selected spatial points windowed with respect to time,
**characterized by** the steps of:
summing (107) all power spectrum; and
determining (108) a spectral peak frequency for said summed power spectra, wherein said determined spectral peak frequency is substantially identical to said heart rate.

8. The computer readable medium according to Claim 7, wherein the method further comprises the step of subtracting (103) a DC offset from each of said selected spatial points.

9. The computer readable medium according to Claim 8, wherein the method further comprises the step of displaying (110) said heart rate.

## Patentansprüche

1. Verfahren zum Bestimmen einer Herzfrequenz aus einem Satz von Ultraschallbildern, umfassend die Schritte:
Erlangen (101) eines Satzes von Ultraschallbildern;
Auswählen (102) eines Teilsatzes räumlicher Punkte aus dem Satz von Ultraschallbildern;
Eintragen jedes der ausgewählten räumlichen Punkte in Bezug auf Zeit;
Anwenden (104) einer Fensterfunktion an einem Fenster jedes der ausgewählten räumlichen Punkte in Bezug auf Zeit; und
Verarbeiten (106) von Daten, die jedem der ausgewählten räumlichen Punkte entsprechen, enthaltend ein Berechnen eines Leistungsspektrums für jeden der ausgewählten räumlichen Punkte, die in Bezug auf Zeit gefenstert sind;
**gekennzeichnet durch** die Schritte:
Summieren (107) aller Leistungsspektren; und
Bestimmen (108) einer spektralen Spitzenfrequenz für die summierten Leistungsspektren, wobei die bestimmte spektrale Spitzenfrequenz im Wesentlichen mit der Herzfrequenz identisch ist.

2. Verfahren nach Anspruch 1, weiter umfassend den Schritt zum Subtrahieren (103) eines DC-Versatzes von jedem der ausgewählten räumlichen Punkte.

3. Verfahren nach Anspruch 1, weiter umfassend den Schritt zum Anzeigen (110) der Herzfrequenz.

4. System (200) zum Bestimmen einer Herzfrequenz aus einem Satz von Ultraschallbildern, umfassend:
Mittel (202) zum Erlangen eines Satzes von Ultraschallbildern;
Mittel (214) zum Auswählen eines Teilsatzes räumlicher Punkte aus dem Satz von Ultraschallbildern;
Mittel zum Eintragen jedes der ausgewählten räumlichen Punkte in Bezug auf Zeit;
Mittel zum Anwenden einer Fensterfunktion an einem Fenster jedes der ausgewählten räumlichen Punkte in Bezug auf Zeit;
Mittel (206) zum Verarbeiten von Daten, die jedem der ausgewählten räumlichen Punkte entsprechen, enthaltend Mittel zum Berechnen eines Leistungsspektrums für jeden der ausgewählten räumlichen Punkte, die in Bezug auf Zeit gefenstert sind;
**dadurch gekennzeichnet, dass** das System (200) weiter enthält:
Mittel (206) zum Summieren aller Leistungsspektren; und
Mittel zum Bestimmen einer spektralen Spitzenfrequenz für die summierten Leistungsspektren, wobei die bestimmte spektrale Spitzenfrequenz im Wesentlichen mit der Herzfrequenz identisch ist.

5. System nach Anspruch 4, weiter umfassend Mittel zum Subtrahieren eines DC-Versatzes von jedem der ausgewählten räumlichen Punkte.

6. System nach Anspruch 5, weiter umfassend Mittel (210) zum Anzeigen der Herzfrequenz.

7. Computerlesbares Medium, das einen Satz programmierbarer Anweisungen speichert, die von einem Prozessor (206) auszuführen sind, um ein Verfahren zum Bestimmen einer Herzfrequenz aus einem Satz erlangter Ultraschallbilder durchzuführen, umfassend die Schritte:
Auswählen (102) eines Teilsatzes räumlicher Punkte aus dem Satz von Ultraschallbildern;
Eintragen jedes der ausgewählten räumlichen Punkte in Bezug auf Zeit;
Anwenden einer Fensterfunktion an einem Fenster jedes der ausgewählten räumlichen Punkte in Bezug auf Zeit;
Verarbeiten (106) von Daten, die jedem der ausgewählten räumlichen Punkte entsprechen, enthaltend ein Berechnen eines Leistungsspektrums für jeden der ausgewählten räumlichen Punkte, die in Bezug auf Zeit gefenstert sind;
**gekennzeichnet durch** die Schritte:
Summieren (107) aller Leistungsspektren; und
Bestimmen (108) einer spektralen Spitzenfrequenz für die summierten Leistungsspektren, wobei die bestimmte spektrale Spitzenfrequenz im Wesentlichen mit der Herzfrequenz identisch ist.

8. Computerlesbares Medium nach Anspruch 7, wobei das Verfahren weiter den Schritt zum Subtrahieren (103) eines DC-Versatzes von jedem der ausgewählten räumlichen Punkte umfasst.

9. Computerlesbares Medium nach Anspruch 8, wobei das Verfahren weiter den Schritt zum Anzeigen (110) der Herzfrequenz umfasst.

## Revendications

1. Procédé de détermination d'une fréquence cardiaque à partir d'un ensemble d'images ultrasoniques, comprenant les étapes consistant à :
acquérir (101) un ensemble d'images ultrasoniques ;
sélectionner (102) un sous-ensemble de points spatiaux à partir dudit ensemble d'images ultrasoniques ;
rapporter graphiquement chacun desdits points spatiaux sélectionnés en fonction du temps ;
appliquer (104) une fonction de fenêtre pour fenêtrer chacun desdits points spatiaux sélectionnés en fonction du temps ; et
traiter (106) des données correspondant à chacun desdits point spatiaux sélectionnés, incluant le calcul d'un spectre de puissance pour chacun desdits points spatiaux sélectionnés fenêtrés en fonction du temps,
**caractérisé par** les étapes consistant à :
sommer (107) tous les spectres de puissance ; et
déterminer (108) une fréquence de pic spectrale pour lesdits spectres de puissance sommés, dans lequel ladite fréquence de pic spectrale déterminée est sensiblement identique à ladite fréquence cardiaque.

2. Procédé selon la revendication 1, comprenant en outre l'étape de soustraction (103) d'une compensation CC de chacun desdits points spatiaux sélectionnés.

3. Procédé selon la revendication 1, comprenant en outre l'étape d'affichage (110) de ladite fréquence cardiaque.

4. Système (200) pour déterminer une fréquence cardiaque à partir d'un ensemble d'images ultrasoniques, comprenant :
des moyens (202) pour acquérir un ensemble d'images ultrasoniques ;
des moyens (214) pour sélectionner un sous-ensemble de points spatiaux parmi ledit ensemble d'images ultrasoniques ;
des moyens pour rapporter graphiquement chacun desdits points spatiaux sélectionnés en fonction du temps ;
des moyens pour appliquer une fonction de fenêtre afin de fenêtrer chacun desdits points spatiaux sélectionnés en fonction du temps ;
des moyens (206) pour traiter des données correspondant à chacun desdits points spatiaux sélectionnés, incluant des moyens pour calculer un spectre de puissance pour chacun desdits points spatiaux sélectionnés fenêtrés en fonction du temps,
**caractérisé en ce que** le système (200) inclut en outre :
des moyens (206) pour sommer tous les spectres de puissance ;
des moyens pour déterminer une fréquence de pic spectrale pour lesdits spectres d'énergie sommés, dans lequel ladite fréquence de pic spectrale déterminée est sensiblement identique à ladite fréquence cardiaque.

5. Système selon la revendication 4, comprenant en outre des moyens pour soustraire une compensation CC de chacun desdits points spatiaux sélectionnés.

6. Système selon la revendication 5, comprenant en outre des moyens (210) pour afficher ladite fréquence cardiaque.

7. Support lisible par ordinateur stockant un ensemble d'instructions programmables à exécuter par un processeur (206) afin d'effectuer un procédé de détermination de la fréquence cardiaque parmi un ensemble d'images ultrasoniques acquises, comprenant les étapes consistant à :
sélectionner (102) un sous-ensemble de points spatiaux à partir dudit ensemble d'images ultrasoniques ;
rapporter graphiquement chacun desdits points spatiaux sélectionnés en fonction du temps ;
appliquer une fonction de fenêtre pour fenêtrer chacun desdits points spatiaux sélectionnés en fonction du temps ; et
traiter (106) des données correspondant à chacun desdits point spatiaux sélectionnés, incluant le calcul d'un spectre de puissance pour chacun desdits points spatiaux sélectionnés fenêtrés en fonction du temps,
**caractérisé par** les étapes consistant à :
sommer (107) tous les spectres de puissance ; et
déterminer (108) une fréquence de pic spectrale pour lesdits spectres de puissance sommés, dans lequel ladite fréquence de pic spectrale déterminée est sensiblement identique à ladite fréquence cardiaque.

8. Support lisible par ordinateur selon la revendication 7, dans lequel le procédé comprend en outre l'étape de soustraction (103) d'une compensation CC de chacun desdits points spatiaux sélectionnés.

9. Support lisible par ordinateur selon la revendication 8, dans lequel le procédé comprend en outre l'étape d'affichage (110) de ladite fréquence cardiaque.
